# EUROPÄISCHE PATENTSCHRIFT

(11) **EP 0 802 766 B1**
(45) Veröffentlichungstag und Bekanntmachung des Hinweises auf die Patenterteilung: **07.07.1999**
(21) Anmeldenummer: 95939235.8
(22) Anmeldetag: 02.11.1995
(51) Int. Cl.: A61B 5/0205, A61B 8/06, A61B 5/0404, A61B 5/0476

(54) **TRAGBARES MEDIZINISCHES MESS- UND DIAGNOSEGERÄT**
PORTABLE MEDICAL MEASUREMENT AND DIAGNOSING APPARATUS
APPAREIL PORTATIF MEDICAL DE MESURE ET DE DIAGNOSTIC

(30) Priorität: 04.11.1994 DE 9418357 U; 31.05.1995 DE 29509013 U
(43) Veröffentlichungstag der Anmeldung: 29.10.1997
(73) Patentinhaber: SCHNEIDER, Edgar, 85386 Eching (DE)
(72) Erfinder: SCHNEIDER, Edgar, D-85386 Günzenhausen (DE); MAUSER, Rudolf, D-65510 Idstein/Taunus (DE)
(74) Vertreter: Winter, Brandl, Fürniss, Hübner, Röss, Kaiser, Polte, Kindermann Partnerschaft
(86) Internationale Anmeldenummer: EP9504306
(87) Internationale Veröffentlichungsnummer: WO9614014

(56) Entgegenhaltungen:
- EP-A- 0 101 870
- WO-A-89/00024
- WO-A-90/00366
- WO-A-92/07515
- DE-A- 2 555 134
- DE-U- 8 813 806
- DE-U- 8 911 113
- US-A- 4 515 164

## Beschreibung

Die Erfindung betrifft ein tragbares medizinisches Meß- und Diagnosegerät gemäß Anspruch 1 sowie ein medizinisches Meß- und Diagnosesystem nach Anspruch 33.

Für die medizinische Diagnostik sind seit längerem Ultraschall-Meßgeräte für die Gefäßdiagnostik bekannt, die nach dem Dopplerprinzip arbeiten. Hierbei handelt es sich hauptsächlich um größere stationäre Geräte mit einer Vielzahl von Möglichkeiten, die jedoch entsprechend teuer sind.

Von der Firma Kretz Technik GmbH wird unter der Bezeichnung Sonos ein tragbares Ultraschall-Meßgerät für die Gefäßdiagnostik vertrieben. Das Gerät ist batteriebetrieben, weist eine optische Anzeigevorrichtung und einen Anschluß für Kopfhörer auf. Die Ultraschallsonde wird über ein Kabel mit dem eigentlichen Gerät verbunden. Für die Bedienung und Handhabung dieses Geräts muß daher mit zwei Händen erfolgen.

Aus DD-A 209 966 ist ein mit einer Hand bedienbares Ultraschallmeßgerät bekannt, das zum Trächtigkeitsnachweis in der Veterinärmedizin dient. Für Messungen der Strömungsrichtung und -geschwindigkeit ist dieses Gerät nicht geeignet.

Aus der DE-OS 38 10 289 ist eine stiftartige Ultraschallsonde bekannt, die mit einer Hand handhabbar ist. Diese bekannte stiftartige Ultraschallsonde ist über ein Kabel mit der Auswerte-, Bedienungs- und Anzeigeeinheit verbunden.

Aus US-A-4 515 164 ist ein mit einer Hand tragbares Ultraschallmeßgerät mit zwei im Abstand voneinander angeordneten Sonden bekannt, das zum Auffinden von Blutgefäßverschlüssen und -verengungen dient. Durch die zwei im Abstand voneinander angeordneten Ultraschallsonden kann die unterschiedliche Fließgeschwindigkeit vor und nach dem Verschluß oder der Verengung erfaßt werden.

Aus WO-A-9 000 366 ist ein Meß- und Diagnosegerät gemäß dem Oberbegriff des Anspruchs 1 bekannt. Dieses bekannte Gerät ist in Form eines Armbandes ausgeführt, wobei die Anzeige und die Auswerteelektronik am Handgelenk oben und die Ultraschallmeßeinrichtung unten in der Nähe der Pulsadern vorgesehen ist. Zusätliche Sensorik wird über Drahtleitungen mit dem Armband verbunden.

Demgegenüber ist es Aufgabe der vorliegenden Erfindung ein tragbares medizinisches Meß- und Diagnosegerät zu schaffen, das vielseitig einsetzbar ist und insbesondere auch für die Notfallmedizin geeignet ist. Weiter ist es Aufgabe der vorliegenden Erfindung ein medizinisches Meß- und Diagnosesystem mit einem solchen Gerät zu schaffen.

Die Lösung dieser Aufgabe erfolgt durch die Merkmale des Anspruchs 1 bzw. 33.

Durch die Kombination einer Ultraschallsonde mit einer Meßeinrichtung für andere physiologische Werte, wie für Körpertemperatur, EEG oder EKG etc., wird ein Gerät geschaffen, das im medizinischen Bereich vielseitig verwendbar ist und insbesondere in der Not- und Unfallmedizin eingesetzt werden kann. Durch die kompakte, langgestreckte und stiftähnliche Bauform mit Dimensionen eines schlanken Diktiergeräts und durch eine entsprechende Anordnung der Bedienelemente wird erreicht, daß das erfindungsgemäße Gerät mit ein und derselben Hand gehalten und betätigt werden kann. Damit ist die Arbeit mit dem erfindungsgemäßen Gerät mit einer Hand möglich.

Die verschiedenen Meßwerte werden vorzugsweise in einer in dem tragbaren Gerät untergebrachten Anzeigevorrichtung zur akustisch und/oder optisch zur Anzeige gebracht (Anspruch 2). Alternativ oder zusätzlich lassen sich die Meßdaten intern in einer elektronischen Speichereinrichtung aufzeichnen (Anspruch 19, auf ein elektronisches Stethoskop geben (Anspruch 15) oder vorzugsweise drahtlos auf eine externe Vorrichtung übertragen (Anspruch 25). Damit ist eine große Flexibilität im Einsatz gewährleistet.

Gemäß einer vorteilhaften Ausgestaltung der Erfindung sind die Ultraschallsonde (Anspruch 3) und/oder die Meßeinrichtung für Temperatur, EKG, EEG, etc. (Anspruch 4) auswechselbar. Dadurch läßt sich die Erfindung an unterschiedliche Einsatzzwecke anpassen.

Gemäß einer vorteilhaften Ausgestaltung umfaßt die Erfindung zwei lösbar miteinander verbundene bzw. verbindbare Bauteile (Anspruch 5 und 6). Damit ist es möglich diejenigen Komponenten, die sowohl für Temperaturmessung, EEG, EKG, unterschiedliche Ultraschallsonden (z. B. für unterschiedliche Frequenzbereiche) benötigt werden in dem einen Bauteil zu integrieren und diejenigen Komponenten die für die unterschiedlichen Meßeinrichtungen unterschiedlich sind in dem anderen Bauteil. Damit wird eine größtmögliche Variabilität und Flexibilität bei geringem Zusatzaufwand erreicht.

Durch eine Steckerkodierung (Anspruch 7) ist kein Umstellen des Geräts auf eine andere Meßeinrichtung erforderlich. Vielmehr "erkennt" das Bauteil mit der Signalauswerteeinrichtung und der Signalausgabeeinrichtung welche Meßeinrichtungen in dem anderen Bauteil enthalten sind und stellt sich selbstätig darauf ein oder um. Durch die Steckercodierbarkeit ist eine einfache und sichere Bedienung gewährleistet.

Durch die vorteilhafte Ausgestaltung der Erfindung mit einer Temperaturmeßeinrichtung (Ansprüche 8 oder 13) läßt sich auf einfache Weise die Hauttemperatur eines Patienten bestimmen. Dies ist insbesondere bei Schockpatienten wichtig. Durch die Mehrfachnutzung der Umhüllung der Ultraschallsonde und/oder der EKG- bzw. EEG-Elektroden wird eine kompakte Bauform ermöglicht.

Durch die vorteilhafte Ausrüstung der Erfindung mit einer Diagnoselampe (Anspruch 14) können Meßpunkte auf dem menschlichen Körper schneller und sicherer aufgefunden werden. Dies ist insbesondere dem ärztlichen Notdienst von Vorteil. Außerdem ist es für den Arzt nicht notwendig eine separate Diagnoselampe mitzuführen, was insbesondere in Notfällen vorteilhaft ist.

Durch den Anschluß für ein elektronisches Stethoskop an die vorliegende Erfindung wird die Akzeptanz der Erfindung verbessert, da die Arbeit mit einem Stethoskop jedem Arzt vertraut ist (Anspruch 15). Außerdem läßt es sich vorteilhaft zum Erkennen des Pulses einsetzen, auch wenn er schwach ist.

Durch das Vorsehen einer Einrichtung zum Bestimmen der Herzfrequenz (Anspruch 16) aus dem durch die Ultraschallmeßeinrichtung ermittelten Frequenzunterschied (Δf) vereinfacht sich die Pulsmessung und außerdem wird sie erheblich genauer.

Durch das Vorsehen einer Einrichtung zum Bestimmen des Winkels zwischen Ausbreitungsrichtung des Ultraschalls und der Strömungsrichtung des Blutes, z. B. einer optischen Meßeinrichtung, (Anspruch 17 und 18) ist eine Messung des absoluten Wertes der Strömungsgeschwindigkeit bzw. eine entsprechende Kalibrierung des Meßgeräts möglich. Durch die Winkelmeßeinrichtung lassen sich außerdem Meßfehler durch unterschiedliche Winkelpositionen automatisch korrigieren.

Durch die vorteilhafte Ausgestaltung der Erfindung mit einer elektronischen Speichereinrichtung (Anspruch 19) ist es möglich Meßdaten zwischenzuspeichern, so daß sie mit nachfolgend aufgenommenen Meßdaten verglichen und weiterverarbeitet werden können. Damit ist beispielsweise auch möglich die zeitliche Entwicklung von Meßdaten darzustellen (Anspruch 20). Damit ist ein unmittelbarer optischer Vergleich von im zeitlichem Abstand aufgenommenen Meßdaten möglich. Auch können damit in vorteilhafter Weise im Körper eines Patienten symmetrisch angeordnete Gefäße nacheinander gemessen und anschließend gemeinsam dargestellt werden können.

Mittels der Echtzeituhr (Anspruch 21) ist es zum einen möglich Zeitmessungen durchzuführen und zum anderen ist es möglich diese Zeitmessung sozusagen auf "Knopfdruck" Meßdaten zuzuordnen. Vorzugsweise geschieht die Zuordnung von Meßdaten und Zeit automatisch, d. h. die Meßdaten werden zusammen mit Datum und Uhrzeit gespeichert (Anspruch 24).

Durch die besonders vorteilhafte Ausgestaltung mit einer Diktiergerätefunktion (Anspruch 22 und 24) ist es dem Arzt möglich unmittelbar während der Messung zu der jeweiligen Messung Anmerkungen und sonstige Diagnosen zu diktieren. Auch Patientenname und sonstige erhebliche Daten lassen sich damit sprachlich fixieren. Damit wird die nachträgliche Auswertung der Meßdaten erheblich verbessert und auch vereinfacht. Durch die Zuordnung von Sprachaufzeichnung und/oder Datum und Uhrzeit wird die Diagnose auch sicherer und überprüfbarer. Die Kombination eines Diktiergeräts mit medizinischen Meß- und Diagnosegeräten ist ganz allgemein sinnvoll und nicht auf tragbare Geräte beschränkt.

Durch die Möglichkeit die mit dem erfindungsgemäßen Gerät gewonnen Informationen und Daten auf eine externe Vorrichtung zu übertragen, lassen sich weitergehende Auswertungen vornehmen. Auch lassen sich die Daten mit anderen Patientendaten in der externen Vorrichtung verknüpfen (Ansprüche 25 bis 27). Bei der externen Vorrichtung handelt es sich vorzugsweise um eine Adapterstation. Auf dieser Adapterstation lassen sich weitergehende Auswertungen realisieren, wie z. B. die Ableitung relevanter Daten aus der Original-Delta-F-Funktion. Die Adapterstation ist wiederum vorzugsweise mit eine Standard-Schnittstelle versehen, um sie mit einem Standard-PC oder einem Drucker verbinden zu können. Damit stehen die Daten auch für Krankenkassenabrechnungen zur Verfügung (Anspruch 28).

Die Verknüpfung mit anderen Patientendaten gestaltet sich besonders einfach, wenn das medizinische Meß- und Diagnosegerät und/oder die Adapterstation bzw. die externe Vorrichtung allgemein mit einer Lese/Schreibeinrichtung für Chipkarten ausgerüstet ist (Anspruch 29 und 34).

Durch Akkus, die in der Adapterstation aufgeladen werden wird auf einfache Weise eine autarke Betriebs- und Einsatzbereitschaft gewährleistet (Anspruch 30).

Durch eine Spannungsüberwachung wird verhindert, daß Meßdaten verloren gehen (Anspruch 31).

Durch das Vorsehen einer Quick-Start-Einrichtung ist beispielsweise eine Sofortdiagnostik in der Kreislaufüberwachung möglich, was insbesondere wieder in der Notfallmedizin von Vorteil ist (Anspruch 32).

Weitere Einzelheiten, Merkmale und Vorteile der Erfindung ergeben sich aus der nachfolgenden Beschreibung anhand der Zeichnung.

Es zeigt:
- Fig. 1: ein Blockschaltbild einer ersten Ausführungsform des tragbaren medizinischen Meß- und Diagnosegeräts gemäß der vorliegenden Erfindung,
- Fig. 2: ein Blockschaltbild der Adapterstation des medizinischen Meß- und Diagnosesystems gemäß der vorliegenden Erfindung,
- Fig. 3: eine perspektivische Darstellung einer beispielhaften Ausführungsform der Erfindung, wobei das zweite Bauteil mit der Ultraschallsonde nicht vollständig auf das erste Bauteil aufgesteckt ist,
- Fig. 4: eine Ansicht der in Fig. 3 gezeigten Ausführungsform von der Seite, wobei die beiden Bauteile vollständig zusammengesteckt sind,
- Fig. 5.: eine schematische Darstellung der in Fig. 3 und 3 gezeigten Ausführungsform mit einem Stethoskop,
- Fig. 6: eine schematische Darstellung des medizinischen Meß- und Diagnosesystems gemäß der vorliegenden Erfindung, und
- Fig. 7a, 7b: ein detailiertes Blockschaltbild des in den Figuren 1 bis 4 gezeigten tragbaren medizinischen Meß- und Diagnosegeräts.

In der nachfolgenden Figurenbeschreibung und in der Zeichnung sind gleiche oder einander entsprechende Teile mit gleichen Bezugszeichen versehen.

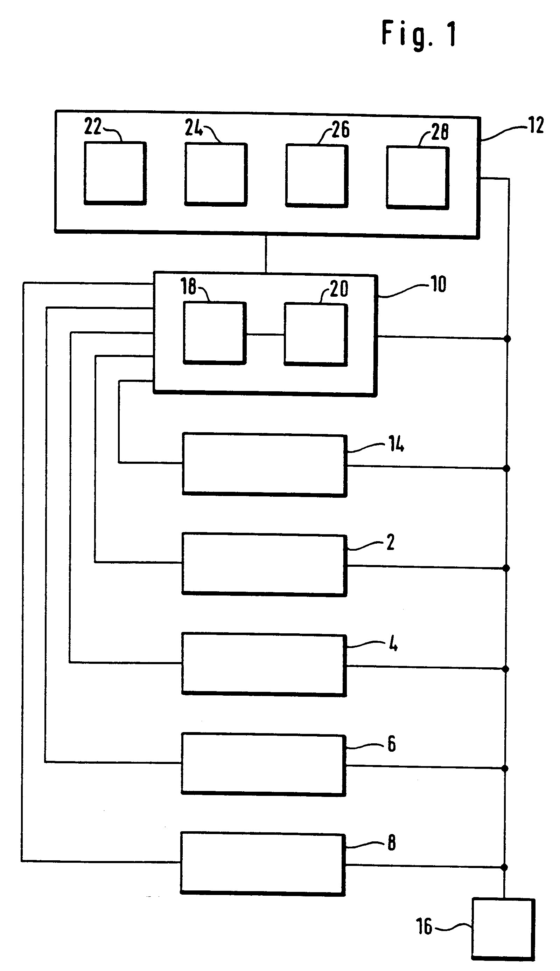

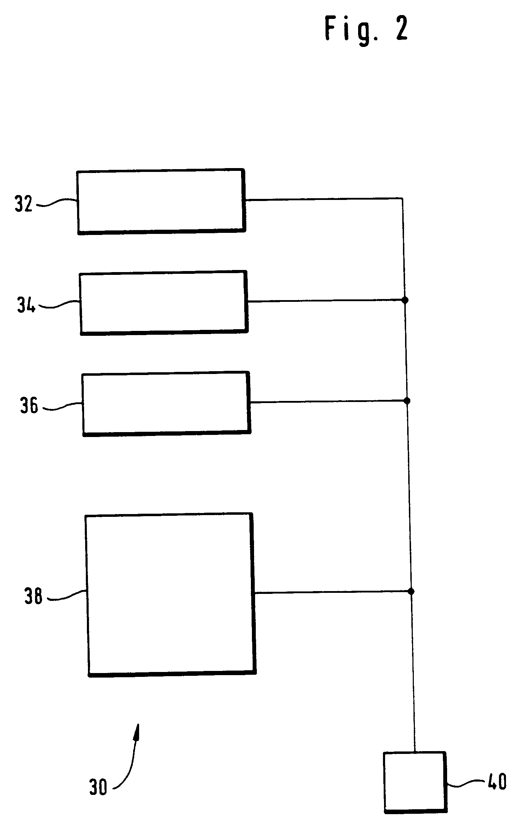

Fig. 1 zeigt ein Blockschaltbild einer ersten Ausführungsform des tragbaren medizinischen Meß- und Diagnosegeräts gemäß der vorliegenden Erfindung. Es umfaßt eine Ultraschall-Meßeinrichtung 2 zur Messung von Strömungsparametern in Blutgefäßen nach dem Dopplerfrequenzprinzip, eine Temperatur-Meßeinrichtung 4, eine EKG- bzw. eine EEG-Meßeinrichtung 6, eine Sprachaufzeichnungseinrichtung 8, eine Signalauswerteeinrichtung 10, eine Ausgabeinrichtng 12 und eine Bedienungseinrichtung 14. Diese einzelnen Komponenten sind mit einer Strom- und Spannungsversorgungseinrichtung 16 verbunden. Die Signalauswertevorrichtung 10 ist mit der Ultraschall-Meßeinrichtung 2, der Temperatur-Meßeinrichtung 4, der EKG- bzw. EEG-Meßeinrichtung 6, der Sprachaufzeichnungseinrichtung 8 und der Ausgabeeinrichtung 12 verbunden. Die Signalauswerteeinrichtung 10 umfaßt einen Mikrocomputer 18 sowie einen elektronischen, digitalen Speicher zum Speichern der anfallenden Meßdaten. Die Ausgabeeinrichtung 12 umfaßt eine graphische Anzeigeeinrichtung 22, z. B. in Form einer LC-Anzeige, eine Lautsprechereinrichtung 24, einem Anschluß 26 für ein elektronisches Stethoskop und eine Infrarot-Schnittstelle 28 zum drahtlosen übermitteln von Daten und Programmen von und zu einer in Fig. 2 als Blockschaltbild dargestellten Adapterstation 30.

Die in Fig. 2 dargestellte Adapterstation 30 umfaßt eine intelligente Ladeelektronik 32 für Akkus 17, eine Infrarot-Schnittstelle 34 für die drahtlose Verbindung zu dem in Fig. 1 dargestellten medizinischen Meß- und Diagnosegerät, eine Lese-Schreibeinrichtung 36 für Chip-Karten mit der insbesondere Daten von und zu den inzwischen üblichen Krankenkassen-Chipkarten übertragen werden können, eine PC-Einheit 38 und eine Strom- und Spannungsversorgung 40. Die PC-Einheit 38 umfaßt alle üblichen Komponenten eines Standard-PC's, wie CPU, Hauptspeicher, Festplatte, Floppy, Eingabetastatur usw.

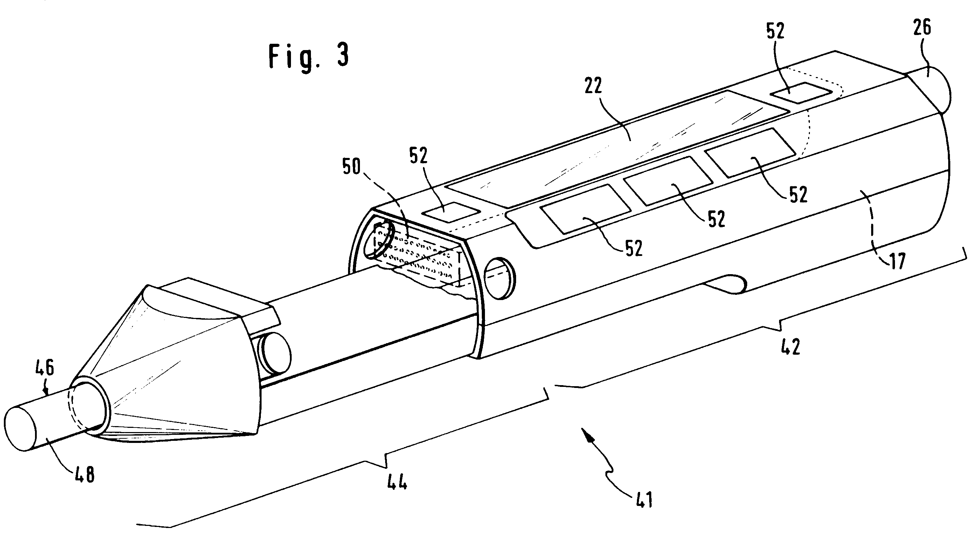

In Fig. 3 ist eine perspektivische Darstellung des in Fig. 1 als Blockschaltbild gezeigten tragbaren medizinischen Meß- und Diagnosegeräts 41 dargestellt. Die in Fig. 1 gezeigten Komponenten sind in dem in Fig. 3 gezeigten Gerät 41 in einem ersten Bauteil 42 und einem zweiten Bauteil 44 untergebracht. Das zweite Bauteil 44 enthält die Ultraschall-Meßeinrichtung 2 und die Temperatur-Meßeinrichtung 4 mit zugehöriger Analog-Elektronik und das erste Bauteil 42 bzw. die Basiseinheit enthält die übrigen Komponenten. An der Spitze des zweiten Bauteils 44 ist die Ultraschall-Messung 46 angeordnet, in dessen Umhüllung auch ein Temperaturfühler 48 der Temperatur-Meßeinrichtung 4 untergebracht ist. Das erste Bauteil 42 und das zweite Bauteil 44 lassen sich zusammenstecken und die elektrische Verbindung wird über einen mehrpoligen Stecker 50 hergestellt. Das zweite Bauteil 44 mit der Ultraschall-Meßeinrichtung 2 wird in verschiedenen Ausfertigungen, z. B. mit 8 MHz, 4 MHz und 2 MHz angeboten.

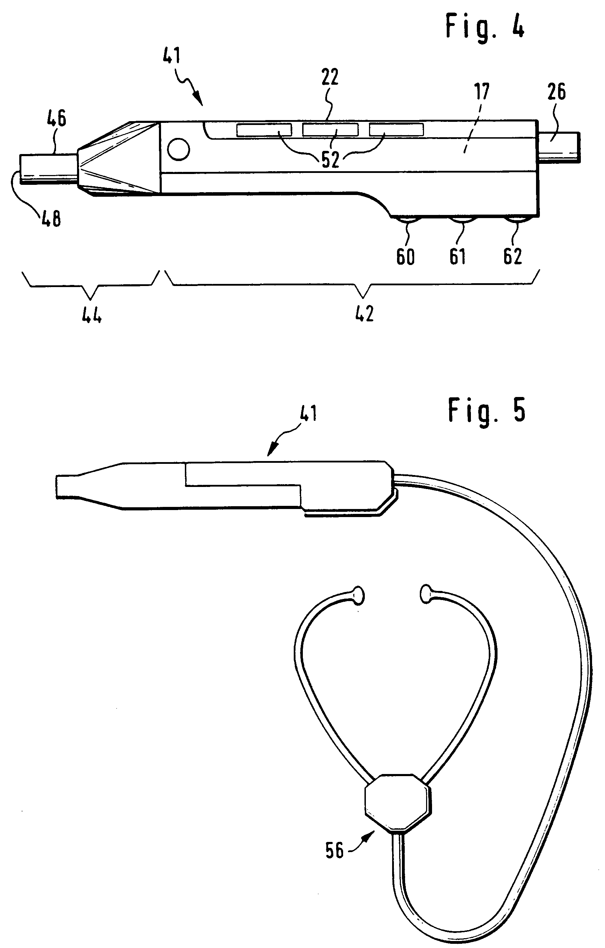

Das erste Bauteil 42 enthält von außen sichtbare Bedienungselemente 52 der Bedienungseinrichtung 14, den Anschluß 26 für ein in Fig. 5 dargestelltes elektronisches Stethoskop 56, die graphische Anzeige 22 und die aus Fig. 4 zu ersehenden Meßelektroden 60, 61 und 62 der EKG- bzw. EEG-Meßeinrichtung 6. Außerdem ist in dem ersten Bauteil 42 noch der Akku 17 untergebracht.

Bei der Anzeigeeinrichtung 22 handelt es sich um eine vollgraphische Flüssigkristall-Matrixanzeige mit einer Auflösung von beispielsweise 320 x 64 Pixeln. Die durch die Ultraschall-Meßeinrichtung 2 ermittelte Blutströmungskurve wird in Echtzeit auf der Anzeige 22 graphisch dargestellt, wobei die Amplitude des dargestellten Signals automatisch auf die Display-Höhe skaliert wird. Auf der Ordinate ist beispielsweise mit 1 bis 2 cm/s der zeitliche Verlauf der Meßkurve dargestellt. Die aufgezeichneten Kurven können nach Beendigung der Messung vorwärts und rückwärts auf der Anzeige 22 "abgefahren" bzw. "gescrollt" werden. Auf der Anzeigeeinrichtung 22 werden neben der Meßkurve auch noch andere Text- und Graphikinformationen dargestellt, die der Kommunikation mit dem Anwender und mit der Adapterstation 30 dienen. Damit läßt sich beispielsweise die Frequenz als Bargraph in kHz darstellen. Die enddiastolische Geschwindigkeit läßt sich als Summenkurve der Flußanteile "vorwärts" und "rückwärts" darstellen.

In der Ausführungsform gemäß Fig. 1 ist der Temperaturfühler 48 der Temperatur-Meßeinrichtung 4 in die Ultraschallsonde 46 integriert. Alternativ kann der Temperatur-Meßfühler 48 auch in eine der Elektroden 60 bis 62 der EKG- bzw. EEG-Meßeinrichtung 6 integriert sein.

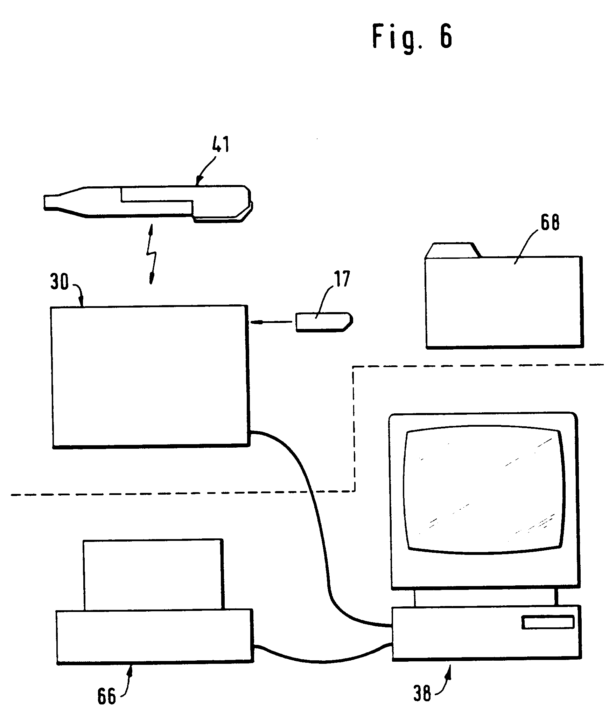

In Fig. 6 ist schematich das medizinische Meß- und Diagnosesystem gemäß der vorliegenden Erfindung mit dem tragbaren medizinischen Meß- und Diagnosegerät 41, wie es in Fig. 1 dargestellt ist, und der Adapterstation 30 mit PC-Einheit 38 dargestellt. Zusätzlich ist in Fig. 6 noch ein an die PC-Einheit 38 angeschlossener Drucker 66 dargestellt. Das in Fig. 6 dargestellte Meß- und Diagnosesystem umfaßt auch noch Anwendersoftware 68 für die Auswertung und Abrechnung der Meßdaten.

Über die in Fig. 3 nicht näher dargestellte Lautsprechereinrichtung 24 werden die Meßgeräusche akustisch dargestellt bzw. über den Anschluß 26 auf das elektronische Stethoskop 56 übertragen. Die Bandbreite reicht von 20 Hz bis 8 kHz im Normalbetrieb. Eine Filterung ist durch Tastendruck realisierbar. Die Tieftonkorrektur ermöglicht eine untere hörbare Grenzfrequenz von wahlweise 20 oder 500 Hz, die Höhenkorrektur eine obere hörbare Grenzfrequenz von 10 oder 2 kHz. Die Lautstärke läßt sich stufenlos am Stethoskop 56 bzw. über eine der Tasten am Bediengerät direkt einstellen.

Die Speichereinrichtung 20 ist beispielsweise als 128 K x 16 SRAM ausgelegt und kann die anfallenden Meßdaten bis zu beispielsweise 30 Minuten aufzeichnen. Über den Mikrocomputer 18 lassen sich zu der jeweiligen Messung Datum und Uhrzeit mitabspeichern. Eine auf der Anzeigeeinrichtung 22 sichtbare bzw. abrufbare "Füllstandanzeige" informiert über den bereits belegten bzw. noch freien Speicher in der Speichereinrichtung 20. Beim Ausschalten des erfindungsgemäßen medizinischen Meß- und Diagnosegeräts 41 bleiben alle Daten in der Speichereinrichtung 20 gespeichert.

Über die Sprachaufzeichnungseinrichtung 8 mit nicht näher dargestellten Mikrofon und Analog-/Digitalwandler kann der Arzt wahrend der Messung Kommentare und Anmerkungen aufzeichnen, die der jeweiligen Messung zugeordnet werden. Die Speicherung der digitalisierten Sprache kann bei entsprechender Kapazitätsauslegung in der Speichereinrichtung 20 erfolgen oder es wird ein zusätzliches Speicherelement in der Sprachaufzeichnungseinrichtung 8 vorgesehen.

Die Infrarotschnittstelle 28 dient zur Kommunikation mit der Adapterstation 30. Es können über die Schnittstelle 28 Meßdaten von dem Handgerät 41 in die Adapterstation 30 überspielt und Befehle und Programme von der Adapterstation 30 in das Handgerät 41 übergeben werden. Der Mikrocomputer 18 des Handgeräts 41 unterstützt den Software-Update mittels Download des Programmcodes aus der Adapterstation 30 zu dem Handgerät 41. Die Energieversorgung des Handgeräts erfolgt über Akkus 17, die in der Adapterstation 30 wieder aufgeladen werden können. Die Energieversorgung von Adapterstation 30 bzw. PC-Einheit 38 erfolgt über das Stromnetz.

Die zum medizinischen Meß- und Diagnosesystem gemäß der vorliegenden Erfindung gehörende Software 68 ermöglicht die Kommunikation mit dem Handgerät 41 (Auslesen von Meßdaten etc.) und das Zuordnen der beispielsweise über die Krankenkassenkarten eingelesenen Patientendaten zu der jeweiligen Messung. Die Meßdatenzuordnung wird durch die Abspeicherung von Datum und Uhrzeit der Messung und zusätzlich durch die der Messung zugeordnete Sprachaufzeichnungen erleichtert. Die Software 68 ermöglicht auch vielfältige graphische Aufbereitungen der Meßdaten und entsprechende Ausdrücke über den Drucker 66.

Durch das Einschalten des Handgeräts 41 über eines der Bedienelemente 52 werden die Elektronik und die Meßeinrichtungen aktiviert. Durch die Betätigung einer weiteren Taste 52 wird der Meßvorgang gestartet, d. h. der Meßvorgang beginnt sofort (Quickstart-Einrichtung). Die Auswahl der Meßart (EKG, EEG, Ultraschall, Temperatur usw. und weitere spezielle Meßparameter) wird über Pulldown-Menüs realisiert. Am Ende der Messung kann bestimmt werden, ob die Daten permanent abgespeichert werden.

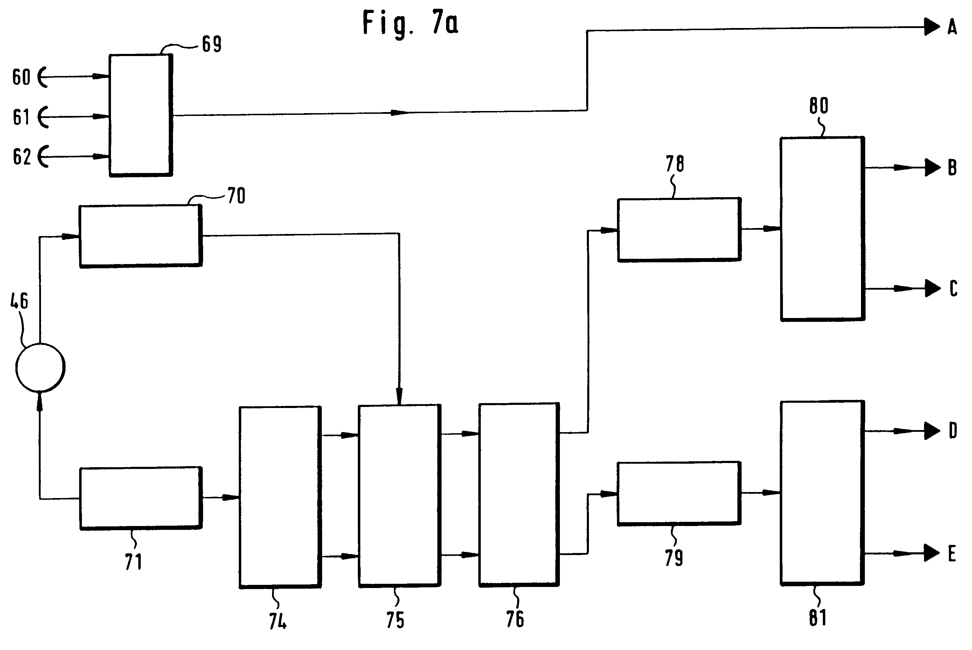

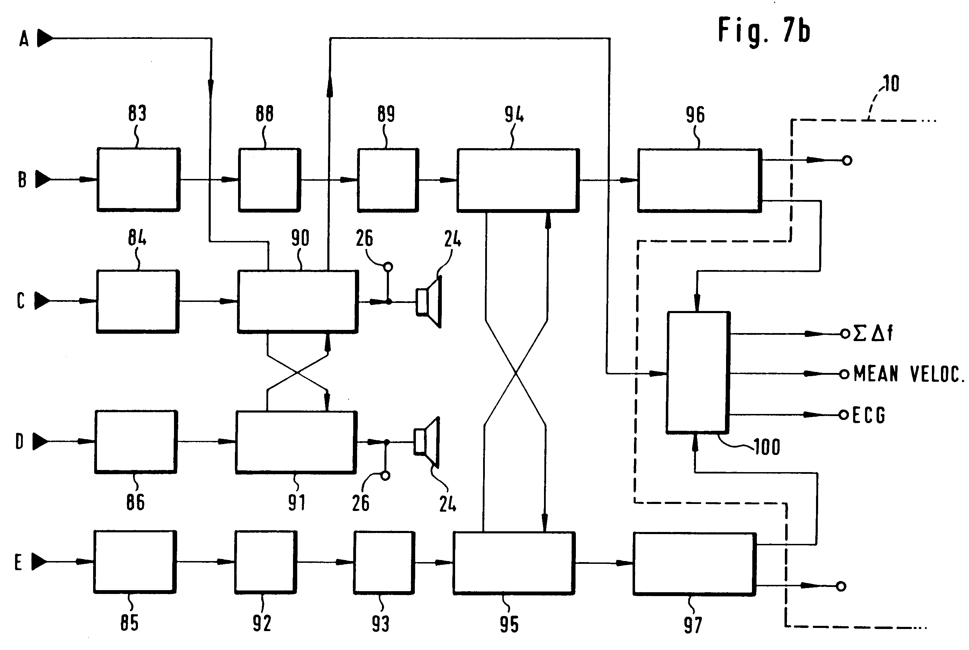

In Fig. 7a und 7b ist ein detailliertes Blockschaltbild der Ultraschall-Meßeinrichtung 2 und der EKG-Meßeinrichtung 6 mit zugehöriger Analogelektronik dargestellt. Die Meßsignale der Elektroden 60, 61, 62 werden in einem Verstärker 69 verstärkt. Die Ultraschall-Sonde 46 umfaßt einen Empfänger 70 und einen Sender 71. Die Signale aus dem Sender 71 werden zum einen von der Ultraschallsonde 46 abgestrahlt und zum anderen einem Phasenschieber 74 zugeführt, der wiederum mit einem Mischer 75 verbunden ist. Die Signale aus dem Empfänger 70 werden ebenfalls dem Mischer 75 zugeführt. Die Signale aus dem Mischer 75 werden einem Phasendetektor 76 zugeführt und gelangen von dort zu einem ersten Demodulator 78 und zu einem zweiten Demodulator 79. Die Signale aus dem ersten Demodulator 78 werden einem ersten Hochfrequenzverstärker 80 zugeführt und die Signale aus dem zweiten Demodulator 79 werden einem zweiten Hochfrequenzverstärker 81 zugeführt.

Die Signale aus dem ersten Hochfrequenzverstärker 80 werden einem ersten Nulldurchgangsdetektor 83 und einem ersten Niederfrequenzfilter 84 zugeführt. Die Signale aus dem Hochfrequenzverstärker 81 werden einem zweiten Nulldurchgangsdetektor 85 und einem zweiten Niederfrequenzfilter 86 zugeführt. Die Signale aus dem ersten Nulldurchgangsdetektor 83 werden einem ersten Verstärker 88 und die Signale aus dem Verstärker 88 werden einem ersten Filter 89 zugeführt. Die Signale aus dem ersten und zweiten Niederfrequenzfilter 84 und 86 werden einem zweiten bzw. dritten Vertärker 90, 91 zugeführt, die miteinander gekoppelt sind. Die Signal aus dem Verstärker 69 werden ebenfalls dem zweiten Vertärker 90 zugeführt. Die Signale aus dem zweiten Nulldurchgangsdetektor 85 werden einem vierten Verstärker 92 und die Signale aus dem vierten Verstärker 92 werden einem zweiten Filter 93 zugeführt. Die Signale aus dem zweiten und dritten Verstärker 90 und 91 werden der Lautsprechereinrichtung 24 bzw. dem Anschluß 26 für ein elektronisches Stethoskop zugeführt. Die Signale aus dem ersten Filter 89 werden einem ersten Phasendetektor 94 und die Signale aus dem zweiten Filter 93 einem zweiten Phasendetektor 95 zugeführt, wobei der zweite Phasendetektor 95 mit dem ersten Phasendetektor 94 gekoppelt ist. Die Signale aus dem ersten Phasendetektor 94 werden einem ersten Ausleseschaltkreis 96 und die Signale aus dem zweiten Phasendetektor 95 einem zweiten Ausleseschaltkreis 97 zugeführt. Die Signale aus den Ausleseschaltkreisen 96 und 97 werden über Anschlüsse 97 direkt der Signalsauswerteeinrichtung 10 zugeführt. Die Signalauswerteeinrichtung 10 umfaßt auch einen schematisch in Fig. 7b dargestellten Schaltkreis 100 zum Bestimmen der Summe der Δf-Signale, der mittleren Strömungsgeschwindigkeit des EKG. Ein Ausgangssignal aus dem Verstärker 90 wird direkt dem Schalkreis 100 zugeführt.

In der Ultraschall-Meßeinrichtung 8 wird durch Δf Verdopplung vor Auswertung ein Detektionsbereich erfaßt, der um den Faktor 2 höher ist als bei herkömmlichen Geräten (MIPM-Technik). In der Auswerteeinrichtung wird diese Verdopplung wieder durch eine entsprechende Kalibrierung wieder kompensiert. Damit lassen sich in vorteilhafter Weise sehr langsame Strömungsgeschwindigkeiten akustisch etc. darstellen.

Mit dem Gerät 41 lassen sich die Blutströmungsverhältnisse in der rechten und linken Körperhälfte auf einfach Weise miteinander vergleichen. Hierzu werden die entsprechenden Messungen in der linken und rechten Körperhälfte nacheinander durchgeführt und gleichzeitig in der Anzeige 22, gegebenenfalls zeitlich versetzt, angezeigt.

Mittels einem in Gehäusekörper des Geräts 41 eingebauten optischen Winkelmessers (nicht dargestellt) ist es möglich die Strömungsgeschwindigkeit in cm/s zu ermitteln und kalibriert darzustellen. Der mit dem Winkelmesser ermittelte Winkel α von Ultraschallsonde 46 zu der Körperoberfläche (Blutgefäß) bzw. zur Strömungsrichtung des Blutes läßt sich ebenfalls in der Anzeige 22 darstellen. Auf der Basis dieses Winkels α ist auch eine mathematische Korrektur der Strömungsgeschwindigkeit in der Auswerteeinrichtng 10 bei Abweichung der Winkelstellung von z.B. 45° möglich.

Die mit der Ultraschallmeßeinrichtung erfaßten und akustisch dargestellten Δf-Signale lassen sich ebenfalls dauerhaft in der Speichereinrichung 20 in digitalisierter Form speichern oder unmittelbar über den den Stethoskopanschluß 26 oder die IR-Schnittstelle 28 auf ein externes Aufzeichnungsgerät übertragen. Damit ist eine Langzeitdokumentation von Meßdaten möglich.

Die Ultraschallsonde 46, der Transducer, läßt sich auch als Katheter ausführen. z. B. mit einer Kathetherlänge von ca. 250 mm und einem Durchmesser von 3-5 mm. Damit kann im Lingualbereich z.B. Tonsilla etc und Gyn. Intrauterin gemessen werden.

In einer medizinischen Notfallsituation muss der agierende Arzt in der kürzest möglichen Zeit eine Diagnose erstellen, um sofort mit der entsprechenden Therapie bzw. Reanimation reagieren zu können. Sind keine entsprechenden Messvorrichtungen vorhanden, kann dies nur rein subjektiv erfolgen und hängt qualitativ von der Erfahrung des Arztes ab. Zwar gibt es in vielen Fällen verschiedene Kriterien, die eine relativ genaue Diagnose zulassen, aber es gibt auch einige, wo dies ohne technische Hilfsmittel nicht möglich ist.

Das Herz kann in derartigen Situationen seine Tätigkeit unterschiedlichst manifestieren, es "schlägt": - normal - zu langsam- arrhythmisch - Stillstand.

Ohne EKG-Gerät sind diese Zustände nur sehr eingeschränkt beurteilbar, zumal bei einem evtl. vorliegenden Herzinfarkt der formale Kurvenverlauf ein besonderes Kriterium darstellt.

Der Blutfluss, und auch der davon abhängige Blutdruck, können hierbei alle möglichen Zustände aufweisen, die ohne entsprechendes Messgerät so gut wie überhaupt nicht beurteilt werden können. Eine weitgehend "normale", rhythmische Herztätigkeit bedeutet nicht gleichzeitig eine korrekte Kreislaufsituation (Kreislaufzentralisation). Mittels Ultraschall kann selbst in schwerem Schockzustand noch eine systolische RR-Determination erstellt werden.

Eine Temperaturmessung ist in vielen Fällen von Vorteil. Bei traumatischen Fällen, etc. ist neben den Kreislaufparametern die Gehirnaktivität - EEG - von entscheidender Bedeutung, z.B. bei Apoplex, rechts-links-Vergleich.

All diese Parameter können mit dem neuen, in der Entwicklung befindlichen Miniaturgerät gemäß der vorliegenden Erfindung registriert werden.

Als typisches Applikationsfeld sei hier u.a. das Flugzeug genannt. Zwar sind mittlerweile viele renommierte Airlines mit Notfallkoffern, sogenannten Doctor-Cases, ausgerüstet; sie enthalten jedoch primär Medikamente und nur relativ selten technisches Gerät. Ein derartiges Miniatur-Diagnosegerät in jedem Verkehrs- und Charterflugzeug würde einen deutlichen diagnostischen Fortschritt und verbesserten medizinischen Service an Bord erbringen und gleichzeitig eine riesige Marktchance eröffnen.

## Patentansprüche

1. Tragbares medizinisches Meß- und Diagnosegerät, mit
einer Ultraschallmeßeinrichtung (2) zur Messung von Strömungsparametern in Blutgefäßen nach dem Dopplerfrequenzprinzip,
wenigstens einer weiteren Meßeinrichtung (4, 6) zur Erfassung anderer physiologischer Meßwerte,
einer Signalauswerteeinrichtung (10) für die von den Meßeinrichtungen gelieferten Meßsignale,
einer Ausgabeeinrichtung (12) für Meßwerte,
einer Bedienungseinrichtung (14), und
einer Strom- und Spannungsversorgung (16),
**dadurch gekennzeichnet,**
daß die einzelnen Komponenten in einer kompakten Bauform (41) integriert sind, die langgestreckt und stiftähnlich ist und die mit ein und derselben Hand gehalten und bedient werden kann.

2. Gerät nach Anspruch 1, dadurch gekennzeichnet, daß die Ausgabeeinrichtung (12) eine graphische und/oder eine akustische Anzeigeeinrichtung (22, 24, 26, 56) zum Anzeigen der von der Signalauswerteeinrichtung (10) erzeugten Signale aufweist.

3. Gerät nach wenigstens einem der vorhergehenden Ansprüche, dadurch gekennzeichnet, daß die Ultraschallmeßeinrichtung (2) auswechselbar ist.

4. Gerät nach wenigstens einem der vorhergehenden Ansprüche, dadurch gekennzeichnet, daß die wenigstens eine Meßeinrichtung für physiologische Meßwerte auswechselbar (4, 6) ist.

5. Gerät nach wenigstens einem der vorhergehenden Ansprüche, dadurch gekennzeichnet, daß das Gerät (41) ein erstes und ein zweites Bauteil (42, 44) umfaßt die mittels einer Steckerverbindung (50) lösbar mechanisch und elektrisch mitteinander verbindbar sind, und daß das erste Bauteil (42) wenigstens die Signalauswerteeinrichtung (10) und das zweite Bauteil (44) wenigstens die Ultraschallmeßeinrichtung (2) umfaßt.

6. Gerät nach Anspruch 5, dadurch gekennzeichnet, daß das erste Bauteil (42) mit unterschiedlichen zweiten Bauteilen (44) verbindbar ist, die unterschiedliche Ultraschallmeßeinrichtungen (2) und/oder unterschiedliche Ultraschallsonden (46) aufweisen.

7. Gerät nach Anspruch 5 oder 6, dadurch gekennzeichnet, daß die zweiten Bauteile (44) steckerkodiert sind.

8. Gerät nach wenigstens einem der vorhergehenden Ansprüche, gekennzeichnet durch eine Temperaturmeßeinrichtung (4) mit einem Temperaturmeßfühler (48).

9. Gerät nach Anspruch 8, dadurch gekennzeichnet, daß der Temperaturmeßfühler (48) ein Kelvin-Sensor ist, der in einem Metallgehäuse gekapselt ist.

10. Gerät nach Anspruch 8 oder 9, dadurch gekennzeichnet, daß der Temperaturmeßfühler (48) in die Spitze der Ultraschallsonde (46) der Ultraschallmeßeinrichtung (2) integriert ist.

11. Gerät nach wenigstens einem der vorhergehenden Ansprüche, gekennzeichnet durch eine EKG-Meßeinrichtung (6), die Meßelektroden (60, 61, 62) umfaßt.

12. Gerät nach wenigstens einem der vorhergehenden Ansprüche, gekennzeichnet durch eine EEG-Meßeinrichtung (6), die Meßelektroden (60, 61, 62) umfaßt.

13. Gerät nach Anspruch 11 oder 12, dadurch gekennzeichnet, daß eine der Meßelektroden (60) als Temperaturfühler (48) ausgebildet ist.

14. Gerät nach wenigstens einem der vorhergehenden Ansprüche, gekennzeichnet durch eine Diagnoselampe.

15. Gerät nach wenigstens einem der vorhergehenden Ansprüche, dadurch gekennzeichnet, daß die Ausgabeeinrichtung (12) einen Anschluß (26) für eine elektronisches Stethoskop (56) aufweist.

16. Gerät nach wenigstens einem der vorhergehenden Ansprüche, dadurch gekennzeichnet, daß die Signalauswertevorrichtung (10) eine Einrichtung (18) zum Bestimmen der Herzfrequenz aus den Meßdaten der Ultraschallmeßeinrichtung (2) umfaßt.

17. Gerät nach wenigstens einem der vorhergehenden Ansprüche, gekennzeichnet durch eine Einrichtung zur Bestimmung des Winkels zwischen Senderichtung der Ultraschallsonde (46) und der Strömmungsrichtung.

18. Gerät nach Anspruch 17, dadurch gekennzeichnet, daß die Signalauswerteeinrichtung (10) eine Einrichtung (18) zum Bestimmen der Strömmungsgeschwindigkeit umfaßt.

19. Gerät nach wenigstens einem der vorhergehenden Ansprüche, gekennzeichnet durch eine elektronische Speichereinrichtung (20) zum Abspeichern von Meßdaten.

20. Gerät nach Anspruch 19, dadurch gekennzeichnet, daß die Ausgabeeinrichtung (12) eine Einrichtung (18, 20, 22) zum gleichzeitigen Darstellen von abgespeicherten und aktuellen Meßwerten in der Anzeigeeinrichtung (22) aufweist.

21. Gerät nach wenigstens einem der vorhergehenden Ansprüche, gekennzeichnet durch eine Echtzeituhr (18).

22. Gerät nach wenigstens einem der vorhergehenden Ansprüche, gekennzeichnet durch eine Einrichtung (8) zum Aufzeichnen von Sprache.

23. Gerät nach Anspruch 22, dadurch gekennzeichnet, daß die Sprachaufzeichnungsvorrichtung (8) mit der elektronischen Speichereinrichtung (20) gekoppelt ist.

24. Gerät nach Anspruch 22 oder 23, gekennzeichnet durch eine Einrichtung (18) zum Zuordnen von Meßdaten zu Sprachaufzeichnungen und/oder Datum und Zeit aus der Echtzeituhr.

25. Gerät nach wenigstens einem der vorhergehenden Ansprüche, dadurch gekennzeichnet, daß die Ausgabeeinrichtung (12) eine Schnittstelleneinrichtung (28) aufweist, mittels der aktuelle oder abgespeicherte Meßdaten auf eine externe Vorrichtung (30, 38) übertragbar sind.

26. Gerät nach Anspruch 25, dadurch gekennzeichnet, daß die Schnittstelleneinrichtung (28) eine Standard-Schnittstelle umfaßt.

27. Gerät nach Anspruchs 25 oder 26, dadurch gekennzeichnet, daß die Schnittstelleneinrichtung (28) eine Einrichtung zum drahtlosen Übertragen von Daten und Befehlen von dem Gerät zu der externen Vorrichtung, insbesondere mittels Infrarotstrahlen, umfaßt.

28. Gerät nach einem der Ansprüche 25 bis 27, dadurch gekennzeichnet, daß die externe Vorrichtung eine Adapterstation (30) ist, die mittels Standard-Schnittstelle mit einem Standard-PC (38) verbindbar ist.

29. Gerät nach wenigstens einem der vorhergehenden Ansprüche, gekennzeichnet durch eine Lese/Schreibeinrichtung für Chipkarten.

30. Gerät nach Anspruch 28 oder 29, dadurch gekennzeichnet, daß die Spannungs- und Stromversorgungseinrichtung (16) auswechselbare Akkus (17) umfaßt, und daß die Adapterstation (30) eine Ladestation (34) für die Akkus umfaßt.

31. Gerät nach wenigstens einem der vorhergehenden Ansprüche, gekennzeichnet durch eine Einrichtung (18) zum Überwachen der Spannungs- und Stromversorgung (16) und zum automatischen Abspeichern der aktuellen Meßdaten und/oder Betriebsparamater, falls die Spannung der Spannungs- und Stromversorgung (16) einen bestimmten Grenzwert unterschreitet.

32. Gerät nach wenigstens einem der vorhergehenden Ansprüche, gekennzeichnet durch eine Quick-Start-Einrichtung durch die das Gerät (41) unmittelbar nach Inbetriebnahme einsatzbereit ist.

33. Medizinisches Meß- und Diagnosesystem mit einem tragbaren medizinischen Meß- und Diagnosegerat (41) nach wenigstens einem der vorhergehenden Ansprüche 25 bis 31 und einer Adapterstation (30), wobei zwischen Meß- und Diagnosegerät (41) und Adapterstation (30) drahtlos Daten austauschbar sind.

34. Medizinisches Meß- und Diagnosesystem nach Anspruch 33, dadurch gekennzeichnet, daß die Adapterstation (30) eine Lese/Schreibeinrichtung für Chipkarten umfaßt.

## Claims

1. A portable medical measuring and diagnostic apparatus comprising
an ultrasound measuring device (2) for measuring flow parameters in blood vessels on the Doppler frequency principle,
at least one other measuring device (4, 6) for detecting other physiological measurements,
a signal evaluation device (10) for the measurement signals delivered by the measuring devices,
an output device (12) for measurements,
an operator control device (14), and
a current and voltage supply (16),
characterised in that
the individual components are integrated in a compact module (41), of elongate and pencil shape, which can be held and operated by just one hand.

2. Apparatus according to claim 1, characterised in that the output device (12) has a graphic and/or an acoustic display device (22, 24, 26, 56) to display the signals generated by the signal evaluation device (10).

3. Apparatus according to at least one of the preceding claims, characterised in that the ultrasound measuring device (2) is interchangeable.

4. Apparatus according to at least one of the preceding claims, characterised in that the at least one measuring device is interchangeable for physiological measurements (4, 6).

5. Apparatus according to at least one of the preceding claims, characterised in that the apparatus (41) comprises a first and second module (42, 44) which by means of a plug connection (50) are electrically interconnectable and mechanically releasable and in that the first module (42) comprises at least the signal evaluation device (10) and the second module (44) comprises at least the ultrasound measuring device (2).

6. Apparatus according to claim 5, characterised in that the first module (42) is adapted to be connected to different second modules (44) having different ultrasound measuring devices (2) and/or different ultrasound probes (46).

7. Apparatus according to claim 5 or 6, characterised in that the second modules (44) are plug-coded.

8. Apparatus according to at least one of the preceding claims, characterised by a temperature measuring device (4) with a temperature measuring sensor (48).

9. Apparatus according to claim 8, characterised in that the temperature measuring sensor (48) is a Kelvin sensor encapsulated in a metal housing.

10. Apparatus according to claim 8 or 9, characterised in that the temperature measuring sensor (48) is integrated in the point of the ultrasound probe (46) of the ultrasound measuring device (2).

11. Apparatus according to at least one of the preceding claims, characterised by an EGG measuring device (6) comprising measuring electrodes (60, 61, 62).

12. Apparatus according to at least one of the preceding claims, characterised by an EEG measuring device (6) comprising measuring electrodes (60, 61, 62).

13. Apparatus according to claim 11 or 12, characterised in that one of the measuring electrodes (60) is constructed as a temperature sensor (48).

14. Apparatus according to at least one of the preceding claims, characterised by a diagnostic lamp.

15. Apparatus according to at least one of the preceding claims, characterised in that the output device (12) has a connection (26) for an electronic stethoscope (56).

16. Apparatus according to at least one of the preceding claims, characterised in that the signal evaluation device (10) comprises a device (18) for determining cardiac frequency from the measurement data from the ultrasound measuring device (2).

17. Apparatus according to at least one of the preceding claims, characterised by a device for determining the angle between the direction of emission of the ultrasound probe (46) and the flow direction.

18. Apparatus according to claim 17, characterised in that the signal evaluation device (10) comprises a device (18) for determining the velocity of flow.

19. Apparatus according to at least one of the preceding claims, characterised by an electronic memory device (20) for storing measurement data.

20. Apparatus according to claim 19, characterised in that the output device (12) comprises a device (18, 20, 22) for simultaneously presenting stored and current measurements in the display device (22).

21. Apparatus according to at least one of the preceding claims, characterised by a real-time clock (18).

22. Apparatus according to any one of the preceding claims, characterised by a device (8) for recording speech.

23. Apparatus according to claim 22, characterised in that the speech recording device (8) is coupled to the electronic memory device (20).

24. Apparatus according to claim 22 or 23, characterised by a device (18) for co-ordinating measurement data with speech recordings and/or date and time from the real-time clock.

25. Apparatus according to at least one of the preceding claims, characterised in that the output device (12) comprises an interface device (28) by means of which current or stored measurement data can be transferred to an external device (30, 38).

26. Apparatus according to claim 25, characterised in that the interface device (28) comprises a standard interface.

27. Apparatus according to claim 25 to 26, characterised in that the interface device (28) comprises a device for wireless transmission of data and commands from the apparatus to the external device, more particularly by infrared rays.

28. Apparatus according to any one of claims 25 to 27, characterised in that the external device is an adapter station (30) which can be connected to a standard PC (38) by a standard interface.

29. Apparatus according to at least one of the preceding claims, characterised by a read/write device for chip cards.

30. Apparatus according to claim 28 or 29, characterised in that the voltage and current supply device (16) comprises replaceable chargeable batteries (17) and in that the adapter station (30) comprises a charging station (34) for the chargeable batteries.

31. Apparatus according to at least one of the preceding claims, characterised by a device (18) for monitoring the voltage and current supply (16) and for automatic storage of the current measurement data and/or operating parameters in the event of the voltage of the voltage and current supply (16) falling below a specific critical value.

32. Apparatus according to at least one of the preceding claims, characterised by a quick-start device by means of the apparatus (41) is immediately ready for use after switching on.

33. A medical measuring and diagnostic system comprising a portable medical measuring and diagnostic apparatus (41) according to at least one of the preceding claims 25 to 31 and an adapter station (30), wherein data can be exchanged by wireless between the measuring device and the diagnostic apparatus (41).

34. A medical measuring and diagnostic system according to claim 33, characterised in that the adapter station (30) comprises a read/write device for chip cards.

## Revendications

1. Appareil médical de mesure et de diagnostic portatif, comportant :
un dispositif de mesure ultrasonore (2) destiné à la mesure des paramètres d'écoulement dans les vaisseaux sanguins selon le principe de la fréquence Doppler,
au moins un autre appareil de mesure (4, 6) destiné à saisir d'autres paramètres physiologiques,
un dispositif d'exploitation de signaux (10) pour les signaux de mesure fournis par les dispositif de mesure,
un dispositif d'édition (12) pour les paramètres,
un dispositif de commande (14), et
une alimentation en courant et tension (16),
caractérisé en ce que les différents composants sont intégrés dans une construction compacte (41) qui est allongée et de type stylo et qui peut être maintenue et actionnée d'une seule et même main.

2. Appareil selon la revendication 1, caractérisé en ce que le dispositif d'édition (12) présente un dispositif d'affichage graphique et/ou acoustique (22, 24, 26, 56) destiné à l'affichage des signaux produits par le dispositif d'exploitation de signaux (10).

3. Appareil selon au moins une des revendications précédentes, caractérisé en ce que le dispositif de mesure ultrasonore (2) est interchangeable.

4. Appareil selon au moins une des revendications précédentes, caractérisé en ce que le au moins un dispositif de mesure des paramètres physiologiques (4, 6) est interchangeable.

5. Appareil selon au moins une des revendications précédentes, caractérisé en ce que l'appareil (41) comporte un premier et un deuxième module (42) qui peuvent être reliés mécaniquement et électriquement de façon amovible au moyen d'un raccord à fiche (50) et en ce que le premier module (42) comporte au moins le dispositif d'exploitation de signaux (10) et le deuxième module (44) au moins le dispositif de mesure ultrasonore (2).

6. Appareil selon la revendication 5, caractérisé en ce que le premier module (42) peut être relié avec différents deuxièmes modules (44) qui présentent des dispositifs de mesure ultrasonores (2) différents et/ou des sondes ultrasonores (46) différentes.

7. Appareil selon la revendication 5 ou 6, caractérisé en ce que les deuxièmes modules (44) ont des fiches codées.

8. Appareil selon au moins une des revendications précédentes, caractérisé par un dispositif de mesure de la température (4) comportant un capteur de mesure de la température (48).

9. Appareil selon la revendication 8, caractérisé en ce que le capteur de mesure de la température (48) est un détecteur de Kelvin qui est encapsulé dans un boîtier métallique.

10. Appareil selon la revendication 8 ou 9, caractérisé en ce que le capteur de mesure de la température (48) est intégré dans la pointe de la sonde ultrasonore (46) du dispositif de mesure ultrasonore (2).

11. Appareil selon au moins une des revendications précédentes, caractérisé par un dispositif de mesure de l'ECG (6) qui comprend des électrodes de mesure (60, 61, 62).

12. Appareil selon au moins une des revendications précédentes, caractérisé par un dispositif de mesure de l'EEG (6) qui comprend des électrodes de mesure (60, 61, 62).

13. Appareil selon la revendication 11 ou 12, caractérisé en ce qu'une des électrodes de mesure (60) est conçue sous forme de capteur de température (48).

14. Appareil selon au moins une des revendications précédentes, caractérisé par une lampe de diagnostic.

15. Appareil selon au moins une des revendications précédentes, caractérisé en ce que le dispositif d'édition (12) comprend une connexion (26) pour un stéthoscope électronique (56).

16. Appareil selon au moins une des revendications précédentes, caractérisé en ce que le dispositif d'exploitation de signaux (10) comporte un dispositif (18) destiné à déterminer la fréquence cardiaque à partir des données de mesure du dispositif de mesure ultrasonore (2).

17. Appareil selon au moins une des revendications précédentes, caractérisé par un dispositif de détermination de l'angle formé entre le sens d'émission de la sonde ultrasonore (46) et le sens de l'écoulement.

18. Appareil selon la revendication 17, caractérisé en ce que le dispositif d'exploitation de signaux (10) comporte un dispositif (18) destiné à déterminer la vitesse d'écoulement.

19. Appareil selon au moins une des revendications précédentes, caractérisé par un dispositif de mémorisation électronique (20) destiné à mémoriser les données de mesure.

20. Appareil selon la revendication 19, caractérisé en ce que le dispositif d'édition (12) présente un dispositif (18, 20, 22) de représentation simultanée de paramètres mémorisés et actuels dans le dispositif d'affichage (22).

21. Appareil selon au moins une des revendications précédentes, caractérisé par une horloge en temps réel (18).

22. Appareil selon au moins une des revendications précédentes, caractérisé par un dispositif (8) d'enregistrement vocal.

23. Appareil selon la revendication 22, caractérisé en ce que le dispositif d'enregistrement vocal (8) est couplé avec le dispositif de mémorisation électronique (20).

24. Appareil selon la revendication 22 ou 23, caractérisé par un dispositif (18) de mise en relation des données de mesure avec les enregistrements vocaux et/ou la date et l'heure provenant de l'horloge en temps réel.

25. Appareil selon au moins une des revendications précédentes, caractérisé en ce que le dispositif d'édition (12) présente un dispositif d'interface (28) au moyen duquel des données de mesure actuelles ou mémorisées peuvent être transférées à un dispositif externe (30, 38).

26. Appareil selon la revendication 25, caractérisé en ce que le dispositif d'interface (28) comprend une interface standard.

27. Appareil selon la revendication 25 ou 26, caractérisé en ce que le dispositif d'interface (28) englobe un dispositif de transmission sans conducteur de données et d'ordres de l'appareil au dispositif externe, notamment par rayonnement infrarouge.

28. Appareil selon une des revendications 25 à 27, caractérisé en ce que le dispositif externe est une station adaptatrice (30) qui peut être reliée à un PC standard (38) au moyen d'une interface standard.

29. Appareil selon au moins une des revendications précédentes, caractérisé par un dispositif de lecture/écriture pour cartes à puce.

30. Appareil selon la revendication 28 ou 29, caractérisé en ce que le dispositif d'alimentation en tension et courant (16) comporte des piles (17) interchangeables et en ce que la station adaptatrice (30) comprend une station de charge (34) pour les piles.

31. Appareil selon au moins une des revendications précédentes, caractérisé par un dispositif (18) de surveillance de l'alimentation en tension et courant (16) et de mémorisation automatique des données de mesure actuelles et/ou des paramètres de fonctionnement, pour le cas où la tension de l'alimentation en tension et courant (16) tombe en dessous d'une valeur limite déterminée.

32. Appareil selon au moins une des revendications précédentes, caractérisé par un dispositif Quick-Start grâce auquel l'appareil (41) est prêt à l'emploi immédiatement après la mise en service.

33. Système médical de mesure et de diagnostic comportant un appareil médical de mesure et de diagnostic portatif (41) selon au moins une des revendications précédentes 25 à 31 et une station adaptatrice (30), des données pouvant être échangées sans conducteur entre l'appareil de mesure et de diagnostic (41) et la station adaptatrice (30).

34. Système médical de mesure et de diagnostic selon la revendication 33, caractérisé en ce que la station adaptatrice (30) comporte un dispositif de lecture/écriture pour cartes à puce.
